# EUROPEAN PATENT SPECIFICATION

(11) **EP 0 494 080 B1**
(45) Date of publication and mention of the grant of the patent: **28.05.1997**
(21) Application number: 92100048.5
(22) Date of filing: 03.01.1992
(51) Int. Cl.: A61B 17/06, A61B 19/02, B65D 75/30, B65D 75/58, B65B 9/02

(54) **Breather pouch for surgical suture packages**
Luftdurchlässiger Beutel für chirurgische Nahtmaterialpackungen
Sachet perméable à l'air pour empaquetages de matériaux de suture chirurgicaux

(30) Priority: 04.01.1991 US 637488
(43) Date of publication of application: 08.07.1992
(73) Proprietor: United States Surgical Corporation, Norwalk, Connecticut 06856 (US)
(72) Inventor: Brown, David L., Wallingford, CT 06492 (US); Malinowski, Stanley J., Guilford, CT 06437 (US)
(74) Representative: Marsh, Roy David

(56) References cited:
- EP-A- 0 322 199
- US-A- 3 163 288
- US-A- 3 357 549
- US-A- 3 419 137
- US-A- 3 926 311
- US-A- 3 954 174
- US-A- 3 991 881
- US-A- 3 995 739
- US-A- 4 365 716

## Description

### BACKGROUND OF THE INVENTION

### 1. Field of the Invention

The present invention relates to packaging devices for surgical elements, and more particularly to breather pouches for enclosing surgical suture-needle packages.

### 2. Discussion of the Prior Art

Breather packages for packaging surgical elements in which a sheet of clear plastic is adhered to a paper, cardboard or other fibrous material backing to provide a sterile display environment for the surgical device are well known in the art. These breather packages generally enclose a sterilized package of surgical devices or elements, such as needles or suture-needle assemblies. The surgical package is fit between the fibrous material backing and the plastic layer and the plastic is then sealed to the fibrous layer to completely enclose the surgical package.

However, while the assembly process of such a package generally provides a secure package which is aesthetically pleasing as a final end product display package, several distinct problems occur upon °pening the package, particularly within the sterile environment of an operating room. One of the more popular packaging materials for use in the surgical suture and surgical instrument field is a material known as Tyvek (a registered trademark of DuPont), which is a fibrous material constructed of spun-bonded polyolefin, in which the polyolefin fibers are spun into a web and compressed to form a high strength porous material. It is common to use Tyvek as the backing material for the breather pouch, whereby the outer layer of plastic material is heat sealed to the edges of the Tyvek sheet to secure the suture package therebetween.

A distinct disadvantage concerning packages formed in this manner, as well as any package including a fibrous material such as Tyvek, cardboard, fiberboard, paper or the like, lies in the fact that when the plastic layer is adhered to the fibrous layer, whether by means of adhesives, heat seals or any other method, upon removing the plastic layer from the fibrous layer there is a tendency for the fibrous layer to separate from itself so that the fibers pull apart from each other. This occurrence, known as "fiber-pull", is similar to a delamination of the layers and occurs at the edges of the fibrous layer where the ends of the fibers are exposed. As the plastic layer is peeled back for removal from the fibrous layer, fiber-pull generally occurs at the edges and results in the continued encapsulation of the package disposed within the breather pouch.

This occurrence of fiber-pull can create two problems in an operating room during surgery. The most important problem is the fact that upon pulling the plastic layer from the fiber layer, if the suture package is still encapsulated in the breather pouch, it requires the surgical team member to further attempt to open the package which may result in a critical delay which may affect the health of the patient. Furthermore, in order to open such a package after the fiber layer has been removed, a sharp instrument may sometimes be necessary to puncture the pouch to get at the package inside. This will require the use of an instrument which has previously been sterilized, and whose sterility may be compromised in order to open the package.

A second problem resulting from the occurrence of fiber-pull is the fact that a large amount of clutter would result in the operating room if a significant number of sutures are required. Again, since the operating room is a sterile environment, the presence of a large number of torn pieces of fibrous paper, as well as particulate fibers which separate from the Tyvek, will be a detriment to the sterile conditions which are required.

In order to overcome the fiber-pull problem, in the prior art there are packages having a Tyvek layer adhered to a plastic layer through the provision of a release agent which is applied to the surface of the Tyvek layer which faces the plastic layer. The entire surface of the Tyvek material is coated with the release agent, which provides for a pull force to separate the plastic from the Tyvek which is lower than the pull force required to separate the fibers from themselves in the Tyvek layer. In general, after the release agent is applied to the Tyvek layer, the surgical suture package is positioned on the Tyvek and then the plastic layer is heat sealed through the release agent to the Tyvek about the periphery of the package.

This construction, while reducing the possibility of fiber-pull and its associated encapsulation problems, also suffers several disadvantages. A significant disadvantage is the increased cost required to produce the breather pouch, since an extensive amount of release agent material is used to cover the entire surface of the Tyvek layer. These release agents generally comprise adhesive materials which adhere to the Tyvek layer and dry to a non-tacky finish.

A second important disadvantage lies in the fact that the application of the release agent to the entire surface of the Tyvek significantly reduces the amount of force necessary to separate the plastic layer from the Tyvek layer. As the plastic layer is peeled back from the Tyvek layer, if too much force is applied the plastic layer completely separates from the Tyvek layer resulting in the possibility that the suture package enclosed therein will fall to the floor. In any event, the complete separation of the plastic layer from the Tyvek layer results in two items to be discarded instead of one, and increases the amount of clutter in the operating room.

A further disadvantage concerns sterilization of the surgical elements packaged within the breather pouch. In general, after the pouch is sealed, the pouch is subjected to a sterilizing gas which passes through the fibrous Tyvek layer. In the prior art, the pouches having adhesive material coating the entire surface of the Tyvek suffer decreased porosity, and accordingly, require longer sterilization times.

A final significant disadvantage lies in the fact that the surgical element may adhere to the adhesive coating during storage, due to storage pressures and temperatures resulting from stacking on shelves or in warehouses. In addition, if the temperatures are too high during the heat sealing process, the surgical element may adhere to the Tyvek layer.

The novel breather pouch for surgical element packages such as sutures and suture-needle assemblies of the present invention obviates the disadvantages encountered in the prior art and provides a breather pouch which substantially reduces or eliminates fiber-pull associated with the prior art. The breather pouch of the present invention overcomes the fiber-pull problem without unduly increasing the cost of the package and further provides a pouch which separates easily to reveal the package enclosed therein without completely separating the plastic layer from the fibrous layer to reduce the amount of clutter in an operating room.

### SUMMARY OF THE INVENTION

The present invention provides a novel breather pouch for packaging and displaying surgical instruments, and in particular suture and suture-needle packages, in which the breather pouch is easily openable through the separation of the upper clear plastic layer from the lower layer of fibrous material. The present invention eliminates the problem of fiber-pull during opening in which the fibrous layer in effect delaminates or separates from itself which results in the continued encapsulation of the packaged suture or suture-needle package between the fibrous material and the plastic cover layer. The present invention also provides a breather pouch which is easily opened but which remains as a single entity even after the suture package is removed therefrom.

The breather package of the present invention essentially comprises a sheet of fibrous material such as Tyvek which is provided with a strip of release agent material along each of its longitudinal edges. A clear plastic sheet corresponding substantially in size to the Tyvek sheet is placed over the Tyvek sheet with a suture package positioned therebetween. The plastic layer is then heat sealed to the Tyvek layer along the longitudinal edges at the release agent material, and is further sealed along the bottom edge directly to the Tyvek material as well as along a top edge which is spaced from the edge of the Tyvek to provide a gripping portion to facilitate separation of the two layers to open the pouch.

Since the transverse top seal is spaced from the edges of the Tyvek and plastic sheets, the possibility of fiber-pull along this edge is essentially eliminated since fiber-pull generally occurs at the edge of the Tyvel. As the top seal is peeled apart, the release agent along the longitudinal edges facilitates opening of the package and substantially eliminates the possibility of fiber-pull along the side edges, by providing a pull-force which is substantially less than the force required to separate the Tyvek fibers from themselves. Also, as a result of the application of the release agent to the side edges, the pull force at the top and bottom seals required to separate the plastic layer from the Tyvek layer is slightly greater than the pull force required to separate the two layers at the side edges. Thus, after the initial pull separates the sheets at the top seal, it is easier to open the rest of the package by pulling the sheets apart since the force required to separate the sheets at the longitudinal edges is less than the force required to separate the initial top heat seal.

As the pouch is completely opened to reveal the suture package enclosed therein, the bottom transverse seal acts as a stop to prevent the plastic layer from completely separating from the Tyvek layer due to the fact that the force required to overcome the bottom seal is now greater than the force required to open the package along the longitudinal side seals. This stop feature significantly reduces the possibility of the suture package falling to the ground as in the prior art, and also eliminates the additional clutter in the operating room by keeping the breather pouch intact as a single discardible unit as opposed to having two pieces to discard.

The present invention is formed on an apparatus which feeds a web of Tyvek material having at least two continuous longitudinally directed strips of release agent material applied thereon at regular intervals starting at the first edge of the web and terminating at the second longitudinal edge. The apparatus feeds the web of Tyvek material and a web of plastic material, preferably polyethylene, to a position to enclose suture packages placed in rows between the plastic and Tyvek layers. This assembly is then fed to a heat seal device which simultaneously provides transverse and longitudinal heat seals to seal the suture packages between the two layers.

Preferably, the plastic web is vacuum formed to provide recesses or pockets to accept the suture packages thereon. The Tyvek then overlays the plastic and suture packages and the heat seal device seal: about the recesses. Alternately, the release agent material may be positioned in longitudinal strips between the Tyvek and plastic layers prior to the heat sealing step, so that the heat seal is through the release agent between the two layers of material. It is preferred, however, that the release agent material be applied directly to the Tyvek layer.

A heat seal platen is applied to the webs to form the seals for adjacent packages. The assembly is then advanced to a cutter mechanism which cuts the pouches just below the transverse seal to form the bottom of one package and along the longitudinal seals, while ensuring that the top edge seals of the individual packages include the gripping tab formed for each package which facilitates separation of the plastic layer from the Tyvek layer to open the pouch.

### BRIEF DESCRIPTION OF THE DRAWINGS

The foregoing features of the present invention will become more readily apparent and may be understood by referring to the following detailed description of an illustrative embodiment of the breather pouch for surgical suture packages and the apparatus for constructing such a package, taken in conjunction with the accompanying drawings in which:
Fig. 1 shows a portion of a web of fibrous material having a release agent material coated thereon as is common in the prior art;
Fig. 2 shows a portion of a web of fibrous material according to the present invention having longitudinal strips of release agent applied thereon;
Fig. 3 illustrates a top plan view of a package for surgical elements according to the present invention;
Figs. 4 and 5 illustrate a plan view of a package according to the present invention in the partially opened and fully opened position, respectively; and
Fig. 6 illustrates a schematic view of an apparatus for assembling the package of the present invention.

### DETAILED DESCRIPTION OF THE PREFERRED EMBODIMENTS

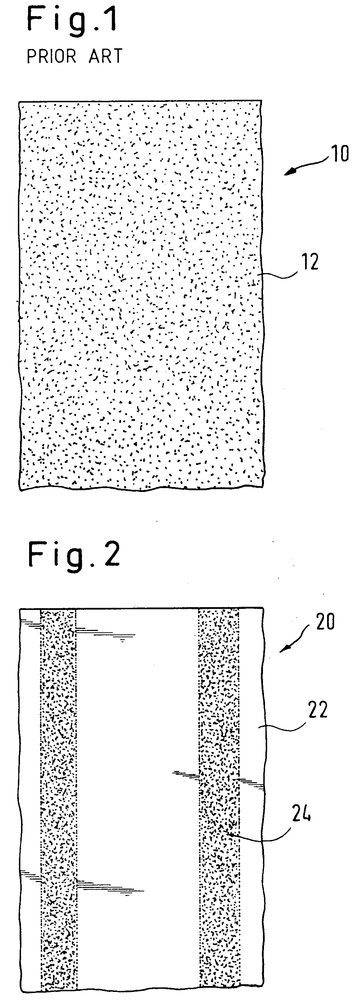

Referring now in specific detail to the drawings, in which like reference numerals identify similar or identical elements throughout the several views, Fig. 1 shows a partial view of a web 10 of fibrous material for use in constructing the package in the prior art. Web 10 is coated over its entire surface with a release agent material 12 which is non-tacky when dry but which covers the entire surface of web 10.

According to the present invention, as best seen in Fig. 2, a web of fibrous material, preferably a spun-bonded polyolefin fiber material such as Tyvek (registered trademark of DuPont), is provided with strips of release agent material 24 applied at regular intervals on surface 22 of web 20. Web 20 is provided with at least two strips of release agent material, and in the preferred form, web 20 is provided with the regularly spaced strips of release agent material 24 beginning at a first longitudinal edge and terminating at a second longitudinal edge of web 20 such that the strips are parallel to each other. The use of web 20 will be described in greater detail below. Web 20 may consist of Tyvek or any other fibrous material such as cardboard, paperboard, or the like.

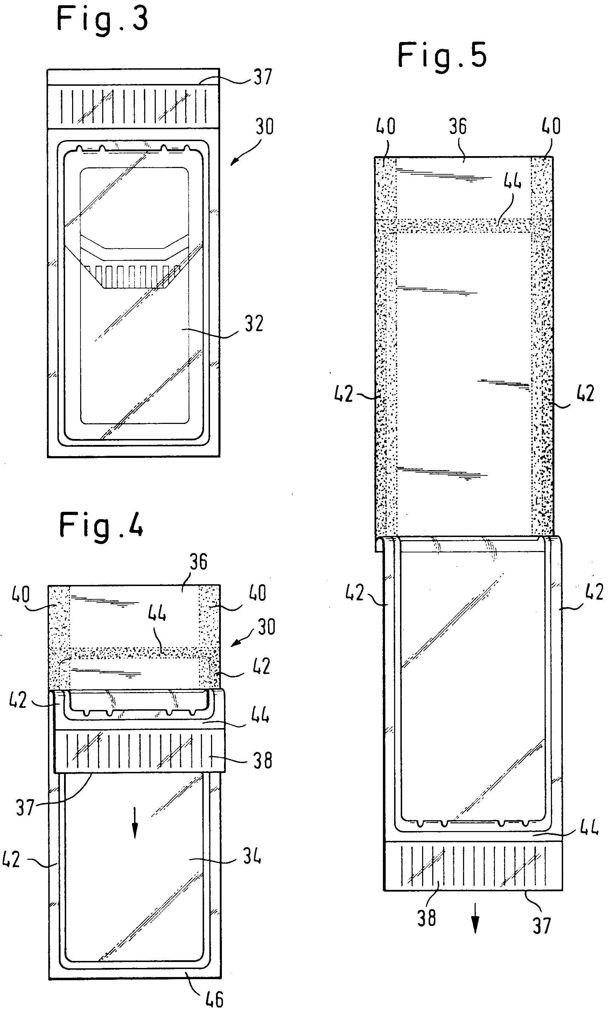

Fig. 3 illustrates a package 30 according to the present invention which essentially comprises a breather pouch which displays a surgical element such as suture package 32. Package 30 essentially comprises a sheet of Tyvek material upon which is positioned surgical element 32 which is then sealed by a cover sheet of plastic material which is heat sealed to the Tyvek to provide a suitable display package.

Figs. 4 and 5 show the package 30 of the present invention with the surgical element 32 removed for purposes of providing clarity to the drawings. Package 30 comprises a sheet of plastic material 34, preferably a polyethylene material, which overlays a sheet of fibrous material 36, which is preferably constructed of Tyvek. Sheet 36 is provided with strips of release agent material 40 which is applied to the longitudinal side edges of sheet 36. Release agent 40 is preferably an adhesive material, such as a water based adhesive, which dries to a non-tacky finish.

After a surgical element such as element 32 is positioned on sheet 36 between strips 40, a sheet of plastic material 34 is positioned in overlying relation to sheet 36 and heat sealed thereto. Plastic sheet 34 is sealed to fiber sheet 36 along longitudinal side seals 42, which seals the plastic sheet 34 to the fibrous sheet 36 at the release agent 40. The package is also sealed by transverse heat seals 44 and 46 which provide a direct seal between the plastic layer 34 and the fibrous layer 36.

In order to open package 30, a gripping tab 38 is provided by positioning top edge seal 44 at a slight distance from the upper edge of the package to allow for ease in gripping the two sheets. Sheet 34 is substantially the same size as sheet 36, but is preferably slightly shorter at top edge 37 to facilitate separation. Fibrous layer 36 is gripped above seal 44, and tab 38 is grasped to pull layer 34 away from layer 36. As the two sheets of material are separated, the surgical element 32 may be removed from the pocket formed therebetween.

The provision of the release agent strips 40 eliminates the possibility of fiber-pull along the longitudinal edges of the package, by providing for a pull force to separate the plastic from the Tyvek which is less than the force required to separate the fibers in the Tyvek from themselves. The force required to separate sheet 34 from sheet 36 at top seal 44 is greater than the force necessary to separate the sheets at seal 42. Thus, after the initial pull to open the package at heat seal 44, it is easier to separate sheet 34 from sheet 36 along longitudinal heat seal 42. On the other hand, since heat seal 46 at the bottom of the package is a seal directly between the plastic layer 34 and the fibrous layer 36, it provides a stop feature to prevent complete separation of the two layers upon opening of the package. Heat seal 46 requires a pull force which is greater than the pull force required to separate sheet 34 from sheet 36 at the longitudinal seals 42.

Preferably, the force required to separate plastic sheet 34 from fibrous sheet 36 at seals 44 and 46 is slightly greater than the force required to separate the sheets at seals 42, so that for the preferred Tyvek-polyethylene pouch the force at seals 44 and 46 may be slightly greater that about 6.9 KPa (1 lb/inch²), for example, between 6.9 and 13.8 KPa (1 lb/inch² and 2 lbs/inch²), while the force at seals 42 is preferably no greater than about 6.9 KPa (1 lb/1 inch²).

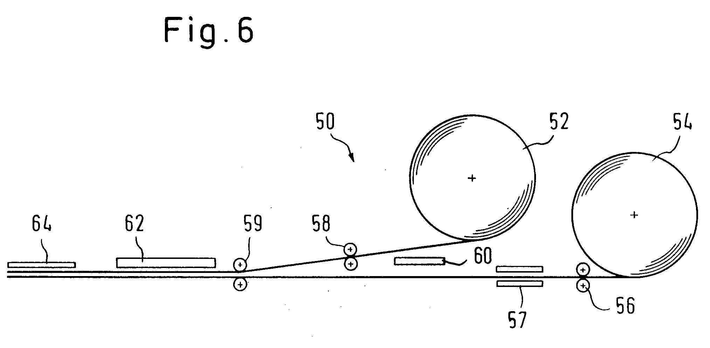

Fig. 6 illustrates an apparatus for assembling package 30 of the present invention. Apparatus 50 provides a web of fibrous material 52 which is similar to the web 20 as shown in Fig. 2, in which at least two longitudinal strips of release agent material are applied thereon. Web 54 comprises a web of plastic material, preferably polyethylene, and web 54 is advanced by advancing means 56 to a vacuum device 57 which forms recesses for accepting surgical elements to be packaged. Surgical elements such as suture or suture-needle assembly packages are placed in spaced relation in the recesses, which are positioned in relation to web 52 between the longitudinal strips of release agent material on web 52 by placement means 60. Web 52 is advanced by advancement means 58 to a heat sealing station along with web 54, and the two webs are then advanced by advancement means 59. Heat sealing station 62 provides longitudinal heat seals which correspond in position to the strips of release agent material, while also providing the transverse heat seal which corresponds to the top and bottom of the packages. The webs are then advanced to cutting station 64 which provides transverse and longitudinal cuts to separate adjacent packages at the transverse and longitudinal heat seals which form the individual packages. The cut at the top seal is preferably made a distance from the seal so as to provide a gripping tab at the top of the package to facilitate opening the package.

While the invention has been particularly shown and described with reference to the preferred embodiments, it will be understood by those skilled in the art that various modifications in form and detail may be made therein without departing from the scope of the invention as defined in the appendant claims. Accordingly, modifications such as those suggested above, but not limited thereto are to be considered within the scope of the invention.

## Claims

1. A peel-apart package (30) for surgical elements comprising:
a sheet (36) of fibrous material having longitudinal side edges and top and bottom transverse edges,
a sheet (34) of plastic material having longitudinal side edges and top and bottom transverse edges, said plastic overlaying and corresponding substantially in size and shape to said fibrous sheet,
a release agent material (40) positioned (40) between said sheets, said plastic sheet being heat sealed (42, 44, 46) to said fibrous sheet along said longitudinal side edges and top and bottom edges, the package being openable by peeling apart progressively the fibrous sheet and the plastic sheet, from the top transverse edge (44) towards the bottom transverse edge (46); **characterized in that**:
i) the release agent is confined to the areas of the longitudinal side edge heat seals (42);
ii) the release agent is absent from the area of the bottom transverse edge heat seal (46); whereby
iii) the release agent preferentially facilitates peeling apart of the fibrous and plastic sheets at the side edges, and the pull force required to separate the sheets at the bottom edges is greater than the pull force required to separate the longitudinal edges.

2. A package according to claim 1, wherein said release agent material is applied as a strip (40) along each longitudinal side edge of said fibrous sheet.

3. A package as claimed in claim 1 or 2 wherein said fibrous sheet is made of cardboard or paper.

4. A package according to any one of the preceding claims, wherein said sheet of fibrous material comprises spun-bonded polyolefin fibers.

5. A package according to any one of the preceding claims wherein said sheet of plastic material comprises polyethylene.

6. A package according to any one of the preceding claims wherein said sheet of fibrous material comprises laminated layers of fibers.

7. A package according to any one of the preceding claims wherein a pull force required to separate said fibrous sheet from said plastic sheet at said transverse top edge is greater than a pull force required to separate said fibrous sheet from said plastic sheet through said release agent at said longitudinal edges.

8. A package according to claim 7 wherein said pull force at said top and bottom edges is between 6.9 to 13.8 KPa (1 and 2 lbs/in²), and said pull force at said longitudinal edges through said release agent is no greater than about 6.9 KPa (1 lbs/in²).

9. A package according to any one of the preceding claims, wherein said release agent comprises an adhesive layer.

10. A package according to any one of the preceding claims, wherein said heat seal at said top edge is spaced from said top edge to define a gripping tab (38) for separating said sheets.

11. A package according to any one of the preceding claims, wherein said surgical elements are within the package, and comprise suture-needle assemblies.

12. Apparatus for packaging surgical elements, said apparatus comprising:
a web (52) of fibrous material in sheet form, said web having a release agent (40) applied thereon and confined to at least two continuous longitudinal strips, said strips beginning at a first longitudinal edge of web material and being spaced at regular identical intervals across said web, said strips ending at a second longitudinal edge of said web;
a web (54) of plastic material having substantially the same width as said web of fibrous material;
means (57) for forming rows of recesses in said plastic material at regular intervals for accepting surgical elements to be packaged;
means (56) for advancing said plastic web;
means (60) for placing said surgical elements at regular intervals in said recesses;
means (58) for advancing said fibrous web into overlying relation with said plastic web, said surgical elements being disposed between said webs, said recesses in plastic web being aligned between said release agent strips;
heat sealing means (62) for heat sealing said plastic web to said fibrous web longitudinally along each of said release agent strips, and for heat sealing said webs transversely from said first longitudinal edge to said second longitudinal edge to enclose each surgical element individually; and
means (64) for cutting said webs at said heat seals to form individual packages.

13. Apparatus according to claim 12, wherein said means (57), for forming recesses in said plastic web comprises a vacuum forming device.

14. A method for packaging surgical elements, said method comprising:
providing a web (52) of fibrous material in sheet form, said web having a release agent (40) applied thereon and confined to at least two continuous longitudinal strips, said strips beginning at a first longitudinal edge of web material and being spaced at regular identical intervals across said web, said strips ending at a second longitudinal edge of said web;
providing a web (54) of plastic material having substantially the same width as said web of fibrous material; forming (57) rows of recesses in said plastic material at regular intervals for accepting surgical elements to be packaged;
advancing (56) said plastic web;
placing 860) said surgical elements at regular intervals in said recesses;
advancing (58) said fibrous web into overlying relation with said plastic web, said surgical elements being disposed between said webs, said recesses in plastic web being aligned between said release agent strips;
heat sealing (62) said plastic web to said fibrous web longitudinally along each of said release agent strips, and heat sealing webs transversely from said first longitudinal edge to second longitudinal edge to enclose surgical element individually; and
cutting (64) said webs at said heat seals to form individual packages.

15. A method according to claim 14, wherein said surgical elements comprise suture-needle asemblies.

16. A method according to claim 14 or 15, wherein said step of forming recesses in said plastic web comprises vacuum forming said recesses.

17. A method according to claim 14, 15 or 16, wherein said transverse top edge seal is spaced from said top edge so as to define a gripping tab (38) to facilitate separation of said plastic layer from said fibrous layer to open said package.

18. A method according to any one of claims 14 to 17, further comprising the steps of applying said release agent to said fibrous web in a plurality of strips, said recesses on said plastic web being positioned between said strips.

19. A method of opening a breather pouch (30) having surgical elements packaged therein, said breather pouch including a sheet (36) of fibrous material having top and bottom transverse edges, and longitudinal side edges; a sheet (36) of plastic material having top and bottom transverse edges and longitudinal side edges, said plastic sheet corresponding substantially in size and shape to said fibrous sheet; a release agent material (24) being positioned (40) between said sheets and confined to each of said longitudinal edges of said sheet; said surgical elements being sealed between said fibrous sheet and said plastic sheet by a transverse heat seal (40) along said bottom edges directly between said plastic and fibrous sheets, a transverse heat seal (44) parallel to and spaced from said top edges to form a fibrous and a plastic gripping tab (38), said transverse heat seal being directly between said plastic and fibrous sheets, and longitudinal heat seals (42) along said side edges which seal said plastic sheet to said fibrous sheet at said release agent whereby the pull force required to separate the sheets at the bottom edge is greater than the pull force required to separate the longitudinal edges; said method comprising the steps of :
grasping said fibrous sheet with one hand at said fibrous gripping tab (36);
grasping said plastic sheet with another hand at said plastic gripping tab (38);
applying a first pull force sufficient to overcome said top transverse heat seal to separate said fibrous sheet from said plastic sheet;
applying a second pull force sufficient to overcome said longitudinal side heat seals to continue separation of said fibrous sheet from said plastic sheet; and
removing said surgical element from said breather pouch.

20. The use, in packaging surgical elements (32), of a web (20) comprising a sheet of fibrous material, and a release agent applied on said sheet and confined to at least two longitudinal strips extending the length of said sheet, said strips beginning at a first longitudinal edge of said sheet and being spaced at regular identical intervals across said sheet, said strips ending at a second longitudinal edge of said sheet.

## Patentansprüche

1. Abziehverpackung (30) für chirurgische Elemente umfassend:
- einen Bogen (36) aus faserförmigem Material mit längs verlaufenden Seitenkanten und oberen und unteren quer verlaufenden Kanten,
- einen Bogen (34) aus Kunststoffmaterial mit längs verlaufenden Seitenkanten und oberen und unteren querverlaufenden Kanten, wobei der Kunststoff über dem faserförmigen Bogen liegt und im wesentlichen bezüglich der Größe und der Form diesem entspricht,
- ein Ablösemittelmaterial (40), das zwischen den Bögen angeordnet (40) ist, wobei der Kunststoffbogen mit dem faserförmigen Bogen entlang der längsverlaufenden Seitenkanten und oberen und unteren Kanten wärmeversiegelt (42, 44, 46) ist, und die Verpackung durch das fortschreitende Auseinanderziehen des faserförmigen Bogens und des Kunststoffbogens von der oberen querverlaufenden Kante (44) in Richtung der unteren querverlaufenden Kante (46) geöffnet wird,
dadurch **gekennzeichnet**, daß
(i) das Ablösemittel auf die Bereiche der längsverlaufenden Wärmeversiegelungen (42) an den Seitenkanten begrenzt ist;
(ii) das Ablösemittel von dem Bereich der Wärmeversiegelung (46) der querverlaufenden unteren Kante abwesend ist; wobei
(iii)das Ablösemittel vorzugsweise das Auseinanderziehen der faserförmigen und Kunststoffbögen an den Seitenkanten erleichtert, und die benötigte Zugkraft, um die Bögen an den unteren Kanten zu trennen, größer ist als die benötigte Zugkraft, um die Längskanten zu trennen.

2. Verpackung gemäß Anspruch 1, wobei das Ablösemittelmaterial als ein Streifen (40) entlang jeder längsverlaufenden Seitenkante des faserförmigen Bogens aufgetragen ist.

3. Verpackung gemäß Anspruch 1 oder 2, wobei der faserförmige Bogen aus Karton oder Papier hergestellt ist.

4. Verpackung gemäß einem der vorhergehenden Ansprüche, wobei der Bogen aus faserförmigem Material gesponnene Polyolefinfasern umfaßt.

5. Verpackung gemäß einem der vorhergehenden Ansprüche, wobei der Bogen aus Kunststoffmaterial Polyethylen umfaßt.

6. Verpackung gemäß einem der vorhergehenden Ansprüche, wobei der Bogen aus faserförmigem Material laminierte Lagen von Fasern umfaßt.

7. Verpackung gemäß einem der vorhergehenden Ansprüche, wobei eine benötigte Zugkraft, um den faserförmigen Bogen von dem Kunststoffbogen an der querverlaufenden oberen Kante zu trennen, größer ist als eine benötigte Zugkraft, um den faserförmigen Bogen von dem Kunststoffbogen durch das Ablösemittel an den Längskanten zu trennen.

8. Verpackung gemäß Anspruch 7, wobei die Zugkraft an den oberen und unteren Kanten zwischen 6,9 und 13,8 kPa (1 und 2 lb/inch²) ist, und die Zugkraft an den längsverlaufenden Kanten durch das Ablösemittel nicht größer als etwa 6,9 kPa (1 lb/inch²) ist.

9. Verpackung gemäß einem der vorhergehenden Ansprüche, wobei das Ablösemittel eine Klebeschicht umfaßt.

10. Verpackung gemäß einem der vorhergehenden Ansprüche, wobei die Wärmeversiegelung an der oberen Kante von der oberen Kante beabstandet ist, um einen Greifvorsprung (38) zum Trennen der Bögen vorzusehen.

11. Verpackung gemäß einem der vorhergehenden Ansprüche, wobei die chirurgischen Elemente innerhalb der Verpackung sind und Nahtmaterial-Nadelzusammenstellungen umfassen.

12. Vorrichtung zum Verpacken von chirurgischen Elementen, wobei die Vorrichtung umfaßt:
- ein Gespinst (52) aus faserförmigem Material in Bogenform, wobei das Gespinst ein Ablösemittel (40) besitzt, das darauf aufgetragen ist und auf zumindest zwei kontinuierliche längsverlaufende Streifen begrenzt ist, wobei die Streifen an einer ersten längsverlaufenden Kante des Gespinstmaterials beginnen und in regelmäßigen gleichen Abständen quer über das Gespinst beabstandet sind, und die Streifen an einer zweiten längsverlaufenden Kante des Gespinstes enden;
- ein Gespinst (54) aus Kunststoffmaterial mit im wesentlichen derselben Breite wie das Gespinst aus faserförmigem Material;
- Mittel (57), um Reihen von Aussparungen in dem Kunststoffmaterial in regelmäßigen Abständen zu bilden, um zu verpackende, chirurgische Elemente aufzunehmen;
- Mittel (56), um das Kunststoffgespinst vorzurücken;
- Mittel (60), um die chirurgischen Elemente in regelmäßigen Abständen in die Aussparungen zu setzen;
- Mittel (58), um das faserförmige Gespinst in einer darüberliegenden Beziehung mit dem Kunststoffgespinst vorzurücken, wobei die chirurgischen Elemente zwischen die Gespinste gesetzt sind und die Aussparungen in dem Kunststoffgespinst zwischen den Ablösemittelstreifen ausgerichtet sind;
- Wärmeversiegelungsmittel (62), um das Kunststoffgespinst an dem faserförmigen Gespinst in Längsrichtung entlang jedes der Ablösemittelstreifen wärmezuversiegeln, und um die Gespinste quer von der ersten längsverlaufenden Kante zu der zweiten längs verlaufenden Kante wärmezuversiegeln, um jedes chirurgische Element einzeln zu umhüllen; und
- Mittel (64), um die Gespinste an den Wärmeversiegelungen zu schneiden, um einzelne Verpackungen zu bilden.

13. Vorrichtung gemäß Anspruch 12, wobei das Mittel (57) zum Bilden von Aussparungen in dem Kunststoffgespinst umfaßt eine Vakuumformeinrichtung.

14. Verfahren zum Verpacken von chirurgischen Instrumenten, wobei das Verfahren umfaßt:
- Vorsehen eines Gespinstes (52) aus faserförmigem Material in Bogenform, wobei das Gespinst ein Ablösemittel (40) besitzt, das darauf aufgetragen ist und auf zumindest zwei kontinuierlich in Längsrichtung verlaufende Streifen begrenzt ist, wobei die Streifen an einer ersten längs verlaufenden Kante des Gespinstmaterials beginnen und in regelmäßigen gleichen Abständen quer über das Gespinst beabstandet sind und an einer zweiten längsverlaufenden Kante des Gespinstes enden;
- Vorsehen eines Gespinstes (54) aus Kunststoffmaterial mit im wesentlichen derselben Breite wie das Gespinst aus faserförmigem Material;
- Bilden (57) von Reihen von Aussparungen in dem Kunststoffmaterial in regelmäßigen Abständen, um zu verpackende chirurgische Elemente aufzunehmen;
- Vorrücken (56) des Kunststoffgespinstes;
- Anordnen (60) der chirurgischen Elemente in regelmäßigen Abständen in die Aussparungen;
- Vorrücken (58) des faserförmigen Gespinstes in eine darüberliegenden Beziehung mit dem Kunststoffgespinnst, wobei die chirurgischen Elemente zwischen die Gespinste angeordnet werden und die Aussparungen im Kunstoffgespinst zwischen den Ablösemittelstreifen ausgerichtet sind;
- Wärmeversiegeln (62) des Kunststoffgespinstes mit dem faserförmigen Gespinstes in Längsrichtung entlang jedes der Ablösemittelstreifen, und
- Wärmeversiegeln der Gespinste in querverlaufender Richtung von der ersten längs verlaufenden Kante zur zweiten längs verlaufenden Kante, um das chirurgische Element einzeln zu umschließen; und
- Schneiden (64) der Gespinste an den Wärmeversiegelungen, um einzelne Verpackungen zu bilden.

15. Verfahren gemäß Anspruch 14, wobei die chirurgischen Elemente Nahtmaterial-Nadelaufbauten umfassen.

16. Verfahren gemäß Anspruch 14 oder 15, wobei der Schritt des Bildens von Aussparungen in dem Kunststoffgespinnst das Vakuumformen der Aussparungen umfaßt.

17. Verfahren gemäß Anspruch 14, 15 oder 16, wobei die Versiegelung der querverlaufenden oberen Kante von der oberen Kante so beabstandet ist, um einen Greifvorsprung (38) zu bilden, um das Trennen der Kunststofflage von der faserförmigen Lage zu erleichtern, um die Verpackung zu öffnen.

18. Verfahren gemäß einem der Ansprüche 14 bis 17 weiter umfassend die Schritte des Anbringens des Ablösemittels an dem faserförmigen Gespinst in einer Mehrzahl von Streifen, wobei die Aussparungen auf dem Kunststoffgespinnst zwischen den Streifen angeordnet sind.

19. Verfahren zum Öffnen einer luftdurchlässigen Tasche (30) mit darin verpackten chirurgischen Elementen, wobei der luftdurchlässige Beutel umfaßt einen Bogen (36) aus faserförmigem Material mit oberen und unteren querverlaufenden Kanten und längsverlaufenden Seitenkanten; einen Bogen (36) aus Kunststoffmaterial mit oberen und unteren querverlaufenden Kanten und längsverlaufenden Seitenkanten, wobei der Kunststoffbogen im wesentlichen bezüglich der Größe und Form dem faserförmigen Bogen entspricht; ein Ablösemittelmaterial (24), das zwischen den Bögen angeordnet (40) ist und auf jede der längs verlaufenden Kanten des Bogens begrenzt ist, wobei die chirurgischen Elemente dicht abgeschlossen sind zwischen dem faserförmigen Bogen und dem Kunststoffbogen durch eine querverlaufende Wärmeversiegelung (40) entlang den unteren Kanten direkt zwischen dem Kunststoff- und faserförmigen Bogen, eine querverlaufende Wärmeversiegelung (44) parallel zu und beabstandet von den oberen Kanten, um einen faserförmigen und einen Kunstoffgreifvorsprung (38) zu bilden, wobei die querverlaufende Wärmeversiegelung direkt zwischen dem Kunststoff und faserförmigen Bogen ist, und längs verlaufende Wärmeversiegelungen (42) entlang den Seitenkanten, die den Kunststoffbogen mit dem faserförmigen Bogen an dem Ablösemittel fest abdichten, wobei die benötigte Zugkraft, um die Bögen an der unteren Kante zu trennen größer ist als die benötigte Zugkraft, um die Längskanten zu trennen; wobei das Verfahren umfaßt die Schritte:
- Greifen des faserförmigen Bogens mit einer Hand an dem faserförmigen Greifvorsprung (36);
- Greifen des Kunststoffbogens mit einer anderen Hand an dem Kunststoffgreifvorsprung (38);
- Anwenden einer ersten Zugkraft, die ausreichend ist, um die obere querverlaufende Wärmeversiegelung zu überwinden, um den faserförmigen Bogen von dem Kunststoffbogen zu trennen;
- Anwenden einer zweiten Zugkraft, die ausreichend ist, um die in Längsrichtung verlaufenden Seitenwärmeversiegelungen zu überwinden, um das Trennen des faserförmigen Bogens von dem Kunststoffbogen fortzusetzen; und
- Entfernen des chirurgischen Elements von dem luftdurchlässigen Beutel.

20. Verwendung beim Verpacken von chirurgischen Elementen (32) eines Gespinstes (20) umfassend einen Bogen aus faserförmigen Material und ein Ablösemittel, das auf dem Bogen aufgetragen ist und auf zumindest zwei längs verlaufende Streifen, die sich entlang der Länge des Bogens erstrecken, begrenzt ist, wobei die Streifen an einer ersten längs verlaufenden Kante des Bogens beginnen und in regelmäßigen gleichen Abständen quer über den Bogen beabstandet sind und die Streifen an einer zweiten längs verlaufenden Kante des Bogens enden.

## Revendications

1. Empaquetage pelable (30) pour des éléments chirurgicaux comportant :
une feuille (36) en matériau fibreux ayant des bords latéraux longitudinaux et des bords transversaux supérieur et inférieur,
une feuille (34) en matière plastique ayant des bords latéraux longitudinaux et des bords transversaux supérieur et inférieur, ledit plastique recouvrant et correspondant sensiblement, en taille et en forme à ladite feuille fibreuse,
un matériau d'agent de relâchement (40) positionné entre lesdites feuilles, ladite feuille en plastique étant thermosoudée (42, 44, 46) à ladite feuille fibreuse le long desdits bords latéraux longitudinaux et bords supérieur et inférieur, l'empaquetage pouvant être ouvert en séparant par pelage progressivement la feuille fibreuse et la feuille en plastique du bord transversal supérieur (44) vers le bord transversal inférieur (46) ;
caractérisé en ce que :
(i) l'agent de relâchement est confiné aux zones des thermosoudures des bords latéraux longitudinaux (42) ;
(ii) l'agent de relâchement est absent de la zone de la thermosoudure du bord transversal de fond (46) ; par quoi
(iii) l'agent de relâchement facilite de préférence une séparation par pelage des feuilles fibreuse et en plastique aux bords latéraux, et la force de traction nécessaire pour séparer les feuilles aux bords de fond est plus grande que la force de traction nécessaire pour séparer les bords longitudinaux.

2. Empaquetage selon la revendication 1, où ledit matériau d'agent de relâchement est appliqué sous forme de bande (40) le long de chaque bord latéral longitudinal de ladite feuille fibreuse.

3. Empaquetage selon la revendication 1 ou 2, où ladite feuille fibreuse est réalisée en carton ou en papier.

4. Empaquetage selon l'une des revendications précédentes, où ladite feuille en matériau fibreux comporte des fibres désorientées en polyoléfine.

5. Empaquetage selon l'une des revendications précédentes, où ladite feuille de matière plastique est constituée de polyéthylène.

6. Empaquetage selon l'une des revendications précédentes, où ladite feuille de matériau fibreux comprend des couches de fibres laminées.

7. Empaquetage selon l'une des revendications précédentes, où une force de traction nécessaire pour séparer ladite feuille fibreuse de ladite feuille en plastique au bord transversal supérieur est plus grande qu'une force de traction nécessaire pour séparer ladite feuille fibreuse de ladite feuille en plastique par ledit agent de relâchement auxdits bords longitudinaux.

8. Empaquetage selon la revendication 7, où ladite force de traction auxdits bords supérieur et inférieur est comprise entre 6,9 et 13,8 KPa (1 et 2 livres/pouce²), et ladite force de traction auxdits bords longitudinaux à travers ledit agent de relâchement n'est pas supérieure à environ 6,9 KPa (1 livre/pouce²).

9. Empaquetage selon l'une des revendications précédentes, où ledit agent de relâchement comprend une couche adhésive.

10. Empaquetage selon l'une des revendications précédentes, où ladite thermosoudure audit bord supérieur est espacée dudit bord supérieur pour définir une patte de préhension (38) pour séparer lesdites feuilles.

11. Empaquetage selon l'une des revendications précédentes, où lesdits éléments chirurgicaux se trouvent dans l'empaquetage et comprennent des ensembles à fils de suture et à aiguilles.

12. Appareil pour emballer des éléments chirurgicaux, ledit appareil comportant :
un tissu (52) en matériau fibreux sous forme de feuille, ledit tissu ayant agent de relâchement (40) appliqué sur celui-ci et confiné à au moins deux bandes longitudinales continues, lesdites bandes commençant à un premier bord longitudinal du matériau de tissu et étant espacées suivant des intervalles réguliers identiques sur ledit tissu, lesdites bandes se terminant à un deuxième bord longitudinal dudit tissu ;
un tissu (54) en matière plastique ayant essentiellement la même largeur que ledit tissu en matériau fibreux ;
un moyen (57) pour former des rangées d'évidements dans ladite matière plastique à des intervalles réguliers pour accepter les éléments chirurgicaux à emballer ;
un moyen (56) pour faire avancer ledit tissu en plastique ;
un moyen (60) pour placer lesdits éléments chirurgicaux suivant des intervalles réguliers dans lesdits évidements ;
un moyen (58) pour faire avancer ledit tissu fibreux en une relation de recouvrement avec ledit tissu en plastique, lesdits éléments chirurgicaux étant disposés entre lesdits tissus, lesdits évidements dans le tissu en plastique étant alignés entre lesdites bandes d'agent de relâchement ;
un moyen de thermosoudage (62) pour thermosouder ledit tissu en plastique audit tissu fibreux longitudinalement le long de chacune desdites bandes d'agent de relâchement et pour thermosouder lesdits tissus transversalement depuis ledit premier bord longitudinal audit deuxième bord longitudinal pour renfermer chaque élément chirurgical individuellement ; et
un moyen (64) pour couper lesdits tissus auxdites thermosoudures pour former des empaquetages individuels.

13. Appareil selon la revendication 12, où ledit moyen (57) pour former des évidements dans ledit tissu en plastique comporte un dispositif de formation à vide.

14. Procédé pour emballer des éléments chirurgicaux, ledit procédé comprenant les étapes consistant à :
réaliser un tissu (52) en matériau fibreux sous forme de feuille, ledit tissu ayant un agent de relâchement (40) appliqué sur celui-ci et confiné à au moins deux bandes longitudinales continues, lesdites bandes commençant à un premier bord longitudinal de matériau de tissu et étant espacées suivant des intervalles réguliers identiques sur ledit tissu, lesdites bandes se terminant à un deuxième bord longitudinal dudit tissu ;
réaliser un tissu (54) en matière plastique ayant sensiblement la même largeur que ledit tissu en matériau fibreux ;
former (57) des rangées d'évidements dans ledit matériau en plastique suivant des intervalles réguliers pour accepter des éléments chirurgicaux à emballer ;
faire avancer (56) ledit tissu en plastique ;
placer (60) lesdits éléments chirurgicaux suivant des intervalles réguliers dans lesdits évidements ;
faire avancer (58) ledit tissu fibreux en une relation de recouvrement avec ledit tissu en plastique, lesdits éléments chirurgicaux étant disposés entre lesdits tissus, lesdits évidements dans le tissu en plastique étant alignés entre lesdites bandes d'agent de relâchement ;
thermosouder (62) ledit tissu en plastique audit tissu fibreux longitudinalement le long de chacune desdites bandes d'agent de relâchement et thermosouder les tissus transversalement depuis ledit premier bord longitudinal au deuxième bord longitudinal pour enfermer l'élément chirurgical individuellement ; et
couper (64) lesdits tissus auxdites thermosoudures pour former des empaquetages individuels.

15. Procédé selon la revendication 14, où lesdits éléments chirurgicaux comprennent des ensembles à fils de suture et à aiguilles.

16. Procédé selon la revendication 14 ou 15, où ladite étape consistant à former des évidements dans ledit tissu en plastique comprend la formation sous vide desdits évidements.

17. Procédé selon la revendication 14, 15 ou 16, où ladite thermosoudure de bord transversale supérieure est espacée dudit bord supérieur de façon à définir une patte de préhension (38) pour faciliter la séparation de ladite couche en plastique de ladite couche fibreuse pour ouvrir ledit emballage.

18. Procédé selon l'une des revendications 14 à 17, comportant en outre les étapes consistant à appliquer ledit agent de relâchement sur ledit tissu fibreux suivant une pluralité de bandes, lesdits évidements sur ledit tissu en plastique étant positionnés entre lesdites bandes.

19. Procédé pour ouvrir un sachet perméable à l'air (30) dans lequel sont emballés des éléments chirurgicaux, ledit sachet perméable à l'air incluant une feuille (36) en matériau fibreux ayant des bords transversaux supérieur et inférieur, et des bords latéraux longitudinaux ; une feuille (36) en matière plastique ayant des bords transversaux supérieur et inférieur et des bords latéraux longitudinaux, ladite feuille en plastique correspond sensiblement en taille et en forme à ladite feuille fibreuse ; un matériau d'agent de relâchement (24) étant positionné (40) entre lesdites feuilles et confiné à chacun desdits bords longitudinaux de ladite feuille ; lesdits éléments chirurgicaux étant scellés entre ladite feuille fibreuse et ladite feuille en plastique par une thermosoudure transversale (40) le long desdits bords de fond directement entre lesdits feuilles en plastique et fibreuse, une thermosoudure transversale (44) parallèle à et espacée desdits bords supérieurs pour former une patte de préhension fibreuse et plastique (38), ladite thermosoudure transversale étant réalisée directement entre lesdites feuilles en plastique et fibreuses, et des thermosoudures longitudinales (42) le long desdits bords latéraux qui soudent ladite feuille en plastique à ladite feuille fibreuse audit agent de relâchement par quoi la force de traction nécessaire pour séparer les feuilles au bord de fond est plus grande que la force de traction nécessaire pour séparer les bords longitudinaux ; ledit procédé comprenant les étapes consistant à :
saisir ladite feuille fibreuse avec une main à ladite patte de préhension fibreuse (36) ;
saisir ladite feuille en plastique avec l'autre main à ladite patte de préhension en plastique (38) ;
appliquer une première force de traction suffisante pour surmonter ladite thermosoudure supérieure transversale pour séparer ladite feuille fibreuse de ladite feuille en plastique ;
appliquer une deuxième force de traction suffisante pour surmonter lesdites thermosoudures longitudinales pour continuer la séparation de ladite feuille fibreuse de ladite feuille en plastique ; et
retirer ledit élément chirurgical dudit sachet perméable à l'air.

20. Utilisation, lors de l'empaquetage d'éléments chirurgicaux (32), d'un tissu (20) comprenant une feuille en matériau fibreux, et un agent de relâchement appliqué sur ladite feuille et confiné à au moins deux bandes longitudinales s'étendant suivant la longueur de ladite feuille, lesdites bandes commençant à un premier bord longitudinal de ladite feuille et étant espacées suivant des intervalles identiques réguliers sur ladite feuille, lesdites bandes se terminant à un deuxième bord longitudinal de ladite feuille.
